# EUROPEAN PATENT APPLICATION

(11) **EP 2 133 149 A1**
(43) Date of publication of application: **16.12.2009**
(21) Application number: 08104411.7
(22) Date of filing: 13.06.2008
(51) Int. Cl.: B01L 3/00

(54) **Lab-on-disc device**

(71) Applicant: F.HOFFMANN-LA ROCHE AG, 4070 Basel (CH); Roche Diagnostics GmbH, 68305 Mannheim (DE)
(72) Inventor: Siljegovic, Vulk, 8932 Mettmenstetten (CH); Jaeggi, Rainer, 8800 Thalwil (CH); Savatic, Goran, 6403 Küssnacht am Rigi (CH); Griss, Patrick, 8112 Otelfingen (CH)
(74) Representative: Poredda, Andreas

(57) **Abstract**

The present invention refers to an analytical device for the analysis of chemical or biological samples comprising a device body, the device body comprising at least one liquid processing unit, the liquid processing unit comprising at least one mixing chamber for mixing at least one sample with at least one reagent, at least one sample dosing chamber for delivering a defined volume of sample to the mixing chamber, at least one reagent channel for delivering at least one reagent to be mixed with the sample, wherein the mixing chamber also serves as detection chamber.

## Description

### Field of the invention

The present invention relates to a microfluidic analytical device for the analysis of chemical or biological samples. The present invention also relates to a method of using such a device and to a system comprising such a device, based on rotation of the device, integrated sample dosing and optical detection.

### Background

There is an enormous need to make diagnostic assays faster, cheaper and simpler to perform while at least maintaining, if not increasing, precision and reliability of conventional laboratory processes.

In order to achieve this goal, substantial effort has been devoted to miniaturization and integration of various assay operations. Conventionally, however, when reaction volumes decrease, other problems increase, such as precise liquid metering, liquid evaporation and problems related to the increased surface to volume ratio. Thus, there is a limit below which it is not possible to go when trying to reduce the scale of a classical state of the art process, typically based on pipetting, mixing and optical detection in cuvettes. When the total assay volume is lowered for example to 50 µL or less, and an even smaller cuvette is used, the following issues start to arise: the precision of the optical path becomes more critical; the robotic handling and positioning of a smaller cuvette is more difficult; evaporation starts to be a concern; the surface forces between liquid and cuvette wall become predominant making the mixing and detection difficult; due to the typical dilution factors, a smaller detection volume means that smaller sample volumes, e.g. below 1 µL would need to be pipetted, and below this range the pipetting precision gets worse dramatically. All these issues make the assay unreliable if at all possible. Among recently developed devices trying to solve these problems are microfluidic devices such as bio-chips and bio-discs.

Gyros AB, Sweden, for example, has developed a compact disk (CD), described e.g. in US 7,275,858 B2, containing several identical application-specific microstructures where samples are processed in parallel, under the control of an automated workstation.

Each microstructure contains integrated functions such as volume metering and packed columns of streptavidin-coated particles. Liquid movement and localization is achieved by a combination of capillary force, centrifugal force and the use of hydrophobic barriers within the microstructure. The CD is intended for heterogeneous immunoassays only. The costs of productions are high. Storage conditions are critical and shelf life is an issue. The analysis procedure is very complex.

Another disc-like device and respective workstation available on the market is that from Abaxis Inc, USA. The Abaxis Laboratory System, consists of a compact, clinical chemistry analyzer for the analysis of electrolytes, blood gas and proteins, using a series of 8-cm diameter single use plastic disc containing the liquid diluents and dry reagents necessary to perform a fixed menu of tests. The disc is placed in the analyzer drawer where centrifugal and capillary forces are used to mix the reagents and sample in the disc.

Also for this system, the costs of productions are high, storage conditions are critical and shelf life is an issue. Moreover, since all the reagents are already present and pre-dosed there is lack of assay flexibility.

An object of the present invention is to provide a simple disposable device and method of using said device which enables reliable and efficient analysis of small volumes of chemical or biological samples. This is achieved by simple liquid processing units in a disc-like device comprising a substrate material and a cover material, enabling precise sample dosing and precise detection in small chambers.

As a consequence the disposable is cheap and storing of reagents with the disposable device can be avoided. Large stocks of devices can be stored without concern for shelf life and storage conditions. Also, the volume reduction achieved by the present invention has the advantage to enable more tests per sample volume, or to run a test when sample availability is limited, e.g. for newborns. Another advantage of the present invention is the reduced consumption of reagents, meaning lower costs per test, more tests per reagent cassette, longer refill times, less waste, lower disposable costs with benefits for the user and the environment. Also, by reducing sample and reagent volumes reactions reach completion more rapidly, thus reducing turnaround time. Another advantage of the present invention is the possibility to use already available test reagents and processes, meaning no cost, time and risk for developing new assays, while maintaining the same test quality. At the same time, assay flexibility is provided, offering the possibility to develop new tests, e.g. for research purposes. Another advantage of the present invention is that, although the device is particularly suitable for clinical chemistry assays, it can be also used for immunoassays.

### Description of the invention

The present invention refers to an analytical device for the analysis of chemical or biological samples comprising a device body, the device body comprising at least one liquid processing unit, the liquid processing unit comprising at least one mixing chamber for mixing at least one sample with at least one reagent, at least one sample dosing chamber for delivering a defined volume of sample to the mixing chamber, at least one reagent channel for delivering at least one reagent to be mixed with the sample, wherein the mixing chamber also serves as detection chamber.

According to the present invention an analytical device is a microfluidic device adapted to carry out various assay operations comprising mixing between liquid samples and reagents as well as detecting the result of those reactions.

According to the present invention, samples are liquid solutions in which one or more analytes of interest can be potentially found. Samples can be chemical and the analytical device can be adapted to carry out one or more chemical assays, e.g. a drug interaction screening, an environmental analysis, the identification of organic substances, etc... Samples can be also biological as e.g. body fluids like blood, serum, urine, milk, saliva, cerebrospinal fluid, etc...

According to a preferred embodiment, the analytical device is adapted to carry out one or more diagnostic assays like e.g. clinical chemistry assays and immunoassays. Typical diagnostic assays include for example the qualitative and/or quantitative analysis of analytes such as albumin, ALP, Alanine Aminotransferase, Ammonia, Amylase, Aspartat Aminotransferase, Bicarbonate, Bilirubin, Calcium, Cardiac Markers, Cholesterol, Creatinine Kinase, D-Dimer, Ethanol, g-Glutamyltransferase, Glucose, HBA1c, HDL-Cholesterol, Iron, Lactate, Lactate Dehydrogenase, LDL-Cholesterol, Lipase, Magnesium, Phosphorus inorganic, Potassium, Sodium, Total Protein, Triglycerides, UREA, Uric Acid. The list is of course not exhaustive.

According to the present invention, the term reagent is used to indicate any liquid, e.g. a solvent or chemical solution, which needs to be mixed with a sample and/or other reagent in order e.g. for a reaction to occur, or to enable detection. A reagent can be for example another sample interacting with a first sample. A reagent can be also a diluting liquid, including water, it may comprise an organic solvent, a detergent, it may be a buffer. A reagent in the more strict sense of the term may be a liquid solution containing a reactant, typically a compound or agent capable e.g. of binding to or transforming one or more analytes present in a sample. Examples of reactants are enzymes, enzyme substrates, conjugated dyes, protein-binding molecules, nucleic acid binding molecules, antibodies, chelating agents, promoters, inhibitors, epitopes, antigens, etc... Optionally dry reagents may be present in the analytical device and be dissolved by a sample, another reagent or a diluting liquid.

According to a preferred embodiment reagents form homogeneous mixtures with samples and the assay is a homogeneous assay. According to another preferred embodiment reagents are heterogeneously mixed with samples and the assay is a heterogeneous assay. An example of heterogeneous assay is a heterogeneous immunoassay, wherein some of the reactants, in this case capturing antibodies, are immobilized on a solid support. Examples of solid supports are streptavidin coated beads, e.g. magnetic beads, or latex beads suspended in solution, used e.g. in latex agglutination and turbidimetric assays.

According to the present invention, the analytical device has a device body comprising at least one liquid processing unit. The device body has preferably the form of a disc, e.g. the footprint of a compact disc (CD). According to one embodiment the device body is a carrier to which one or more liquid processing units can be coupled, and has e.g. the form of a flat rotor which is fixed or can be fixed to a rotatable pin. The term coupled to is here used to indicate that the device body and the liquid processing units are separate entities joined with each other at the moment of use. In this case the device body could be made of a rigid material, e.g. metal, such as aluminum, or a plastic material, e.g. injection molded, and have functional features such as e.g. compartments to receive liquid processing units, alignment pins and/or holes, clamps, levers, or screws to fix the liquid processing units. The device body may have holes enabling optical detection or may even be transparent.

According to a preferred embodiment the device body and the at least one liquid processing unit form one integral piece, made e.g. of a plastic material, preferably injection molded. The device body is preferably at least partially transparent. According to a preferred embodiment the device body is disposable.

A liquid processing unit is either a separate element that can be coupled to the device body, or an integral part of the device body, comprising interconnected microfluidic structures by which it is possible to achieve miniaturization and integration of the various assay operations. The term integral is here used to indicate that the liquid processing unit is at least partially built in the device body at the moment of production and is not separable from the device body.

A liquid processing unit comprises at least two layers, one substrate layer and one cover layer. The microfluidic structures are created preferably on the upper surface of the substrate and sealed from the top with the cover layer. According to one embodiment, the substrate layer is the device body. According to another embodiment, the substrate layer is a separate element that can be coupled to the device body. The cover layer can be made of the same material as the substrate layer or of a different material such as e.g. a thin polymeric foil, preferably transparent. A preferred way of achieving bonding between the substrate layer and the cover layer is thermal bonding. Terms like upper and top are here used as relative and not absolute. The position of substrate layer and cover layer can be for example reversed. The cover layer comprises preferably holes or access ports to enable the access of liquids such as samples, reagents and/or air to the microfluidic structures. A liquid processing unit according to the present invention allows at least one sample to be dosed, to come subsequently in contact with at least one reagent and finally to detect at least one analyte of interest after the at least one sample has been mixed with the at least one reagent.

Different liquid processing units may be partially interconnected between them, e.g. one access port might be in common to more than one liquid processing unit.

According to the present invention the liquid processing unit comprises at least one sample dosing chamber. A sample dosing chamber is a microfluidic structure defined as a cavity between the substrate layer and the cover layer, the volume of which defines the volume of sample to be used in the assay once it has been filled with the sample. Said volume is typically below 1 µL, preferably about 200 nL. The sample dosing chamber has preferably an elongated shape and has at least two microchannels connected to it: one sample inlet channel allowing sample to fill the sample dosing chamber; one liquid decanting channel, defining where the sample dosing chamber starts and the sample inlet channel ends, and allowing excess sample to be guided to a waste chamber. At about the opposite side, the sample dosing chamber comprises a microfluidic valve. Different categories of microfluidic valves are known in the art but all have the same function: temporarily stop the flow of liquid at the point where it is located. According to a preferred embodiment the microfluidic valve is a geometric valve based on changes in the geometrical surface characteristics and thus surface energy. One way of realizing this type of valve is by a restricted conduit ending blunt at the inner edge of a larger channel or chamber. Another type of valve that could be used is based on changes in the chemical surface characteristics resulting also in changes of surface energy. One way to realize this type of valve is e.g. by hydrophobic patterning on hydrophilic surface. Although these types of valves are physically open, the surface energy at this position is such that the force driving the liquid needs to be increased in order to let the liquid flow through the valve. Maintaining the driving force below that required to break the energy barrier of the valve will cause the liquid to stop at this position and any excess to be deviated to the decanting channel characterized by having a barrier energy lower than that of the valve. According to a preferred embodiment the liquid driving force is centrifugal force acting on the liquids by rotating the analytical device. Thus, the movement of liquids inside the liquid processing units is controlled by controlling the speed of rotation of the device body.

According to a preferred embodiment a plurality of liquid processing units are symmetrically arranged around a central axis of rotation of the device body.

According to the present invention, the liquid processing unit comprises at least one mixing chamber for mixing at least one sample with at least one reagent. The mixing chamber is a microfluidic structure defined as a cavity between the substrate layer and the cover layer, defining a lower wall and upper wall respectively, and delimited by side walls. The volume of the mixing chamber defines the maximum volume of reaction mixture. Said volume is typically below 50 µL.

The at least one mixing chamber is communicating with the at least one dosing chamber at least via the valve. According to a preferred embodiment a sample delivery channel extending from the valve to the mixing chamber delivers the sample dosed by the sample dosing chamber to the mixing chamber.

According to the present invention the liquid processing unit comprises at least one reagent channel for delivering at least one reagent to be mixed with the sample. The at least one reagent channel may deliver the at least one reagent to the at least one mixing chamber directly or via an intersecting channel which leads to the mixing chamber, e.g. via the sample delivery channel.

According to the present invention the liquid processing unit further comprises at least one reagent inlet chamber connected to the at least one reagent channel for introducing a defined volume of at least one reagent. Reagents are introduced into the reagent inlet chambers preferably via an access port or hole by means of a pipetting unit.

According to one embodiment a plurality of reagents is introduced sequentially or in parallel to be mixed with the sample. According to one embodiment the same reagent inlet chamber and/or the same reagent channel can be used for a plurality of reagents. According to another embodiment, different reagent inlets and different reagent channels can be used for different reagents. According to another embodiment one reagent inlet chamber is used to distribute at least one reagent to different liquid processing units.

According to a preferred embodiment the liquid processing unit further comprises at least one sample inlet chamber connected to the at least one sample inlet channel for introducing a defined volume of at least one sample. Samples are introduced into the sample inlet chambers preferably via an access port or hole by means of a pipetting unit.

According to one embodiment, one sample inlet chamber is used to distribute at least one sample to different liquid processing units.

According to the present invention the mixing chamber also serves as detection chamber. This means that the presence and/or quantitation of any analyte of interest is determined directly in the mixing chamber after or during the mixing between the at least one sample and the at least one reagent. Detection is typically optical detection, e.g. based on photometric methods such as absorbance measurement, turbidimetry, luminescence, bioluminescence, chemiluminescence, fluorescence, phosphorescence.

In order to enable optical detection, the mixing chamber is made at least partially of a transparent material.

Absorbance measurement can be in-plane or out-of-plane. Out-of-plane detection is characterized by incident light passing through the device body nearly perpendicular to the plane of the device body, e.g. the incident light is coming from the bottom through the device body and/or trough the substrate layer of the liquid processing unit and so through the mixing chamber while a detector is positioned on the opposite side of the cover layer. In-plane detection is characterized by the incident light being reflected by Total Internal Reflection (TIR) or by a mirror-like surface, e.g. a metal coating or a dielectric mirror, integrated with the analytical device and positioned just at the side of the mixing chamber, so that light is passing through the mixing chamber in a direction nearly parallel to the plane of the device body. The detector can in this case be positioned either radially outwards of the device body at nearly 90 degrees from the incident light or on either side, bottom or top, of the analytical device in case, by a similar mechanism, light is reflected at the opposite side of the mixing chamber perpendicularly out of the device body.

The dimension of the mixing chamber in direction of the optical path of the light, i.e. optical path length, needs to be reproducible, especially for absorbance and turbidimetry measurements. According to a preferred embodiment a light beam is guided principally perpendicular to the disc. Preferably the optical read-out is performed on-the-fly, i.e. during rotation of the disc. The light beam has to be shaped in that way that the beam diameter (if circular) or dimension (if deviating from a disc shape), is smaller than the surface of the lower and upper walls of the mixing chamber. In order to avoid distortion or misalignment of the light beam and to guarantee a reproducible/defined optical path, the upper and lower walls of the mixing chamber are preferably perpendicular to the light beam and parallel to each other. In case of in-plane detection, in the vicinity of the side walls, there may be reflective surfaces or edges, e.g. forming an angle of 45° relative to the plane of the device, and deflecting light to an angle of 90° through the plane of the device. The material comprising the mixing chamber through which the light beam is guided is transparent to electromagnetic radiation between about 300 nm and about 1000 nm, preferably between about 300 nm and 850 nm. According to a preferred embodiment, the analytical device is so manufactured that surface scratches and defects at least along the optical path are minimized. Preferably, the optical transmission through the mixing chamber, when it contains a blank solution, is higher than 80 % for the spectral region between 300 nm and 1000 nm (blank measurement). The analytical device or at least the mixing chamber is made from a material fulfilling these optical requirements. Typically plastic materials such as polymethylmethacrylate (PMMA) or acrylate derivatives are used. Alternatively also various glass-like or crystal materials may be used.

According to a preferred embodiment the liquid processing unit further comprises a plasma separation chamber for separating plasma from whole blood.

Plasma separation chambers are known in the art. A microfluidic plasma separation chamber is so designed that under the action of centrifugal force, whole blood gradually enters the chamber from one side; the corpuscolar component of the blood is forced to concentrate towards the outer edge of the chamber facing radially outwards; the plasma liquid component gradually grows in the inner portion of the chamber facing towards the center of the device; when the plasma reaches a certain level, it flows into a collection channel.

According to the present invention said plasma separation chamber precedes in flow direction the sample dosing chamber.

The present invention also refers to a method for the analysis of chemical or biological samples comprising the steps of
a) providing an analytical device comprising a device body, the device body comprising at least one liquid processing unit, the liquid processing unit comprising
   - at least one mixing chamber for mixing at least one sample with at least one reagent, the at least one mixing chamber being at least partially transparent,
   - at least one sample dosing chamber for delivering a defined volume of sample to the mixing chamber,
   - at least one reagent channel for delivering at least one reagent to be mixed with the sample,
   - at least one waste chamber,
b) introducing into said analytical device a chemical or biological sample to be analyzed,
c) rotating the analytical device at a rotational speed so that the sample dosing chamber is filled with the volume of sample to be analyzed while an excess of sample is guided to the waste chamber,
d) increasing the rotational speed to let the sample in the dosing chamber pass into the mixing chamber,
e) introducing at least one reagent into said analytical device,
f) rotating the analytical device at a rotational speed so that the at least one reagent is guided into the mixing chamber,
g) optically detecting through the at least partially transparent mixing chamber the result of the reaction between sample and the at least one reagent.

The total number of steps and the appropriate sequence of steps depend of course on the particular assay. Also, the number as well as the volume of reagents are dependent on the particular assay.

According to a preferred embodiment the method further comprises the step of separating plasma from a blood sample via a plasma separation chamber preceding in flow direction the sample dosing chamber.

The present invention also refers to a system for the analysis of chemical or biological samples comprising
- an analytical device comprising a device body, the device body comprising at least one liquid processing unit, the liquid processing unit comprising at least one mixing chamber for mixing at least one sample with at least one reagent, at least one sample dosing chamber for delivering a defined volume of sample to the mixing chamber, at least one reagent channel for delivering at least one reagent to be mixed with the sample, wherein the mixing chamber also serves as detection chamber,
- a rotor for rotating said analytical device,
- a reagent rack for receiving reagent containers,
- a sample rack for receiving sample containers,
- at least one pipetting unit for introducing samples and/or reagents into said analytical device,
- an optical detection unit for detecting in the mixing chamber the result of the reaction between sample and the at least one reagent.

More in detail, the present invention is explained in conjunction with the following drawings, representing preferred embodiments, in which:
Figure 1 is an exploded view of a liquid processing unit.
Figure 2 is an enlarged top view of the area of figure 1 in correspondence of the dosing chamber.
Figure 3 shows schematically an analytical device comprising a plurality of liquid processing units as those of figure 1 coupled to the device body.
Figures 4 shows schematically an analytical device comprising a plurality of liquid processing units as those of figure 1 integrated with the device body.
Figure 5a is a variant of the liquid processing unit of figure 1 adapted for in-plane detection.
Figure 5b is a side view of the liquid processing unit of figure 5a showing the arrangement of optical structures which enable in-plane-detection.
Figure 6 shows schematically a system comprising the analytical device and means for operating the analytical device.

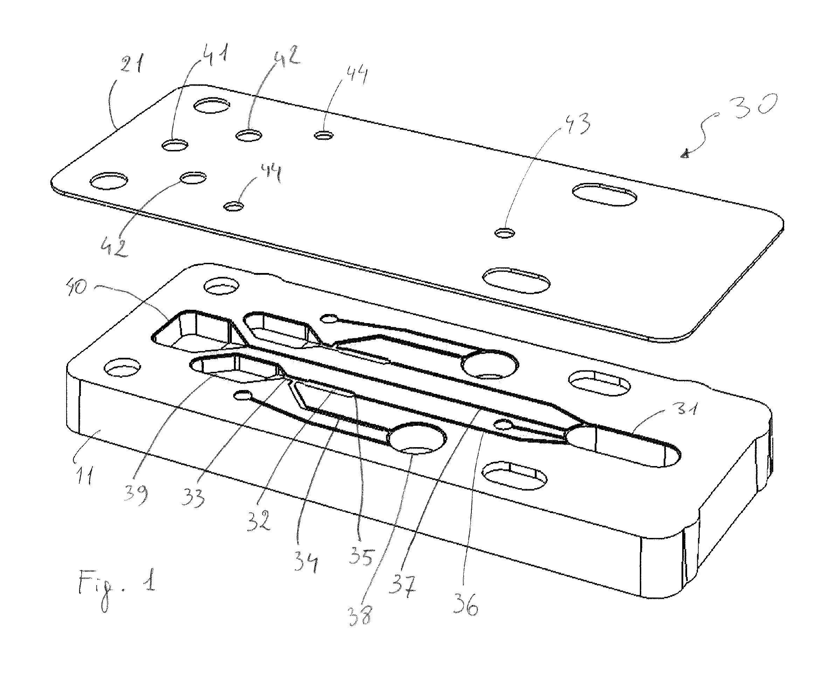

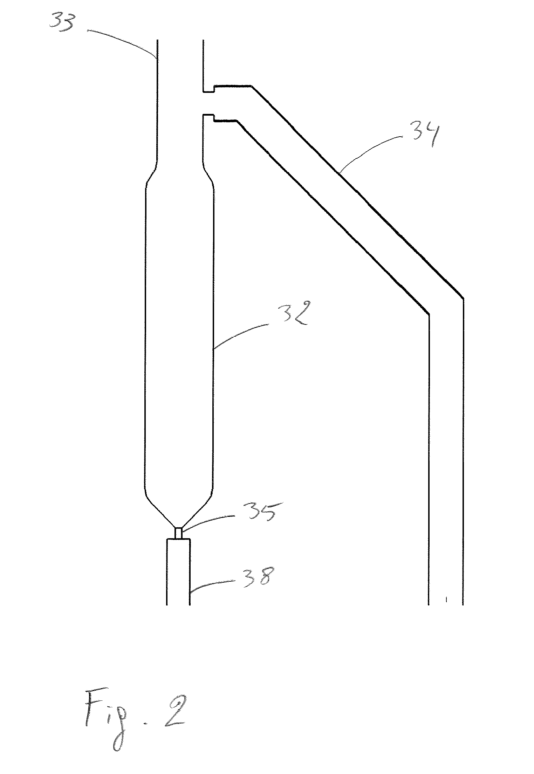

Figure 1 shows an example of liquid processing unit 30, comprising a substrate layer 11 and a cover layer 21, shown for clarity in exploded view. In an assembled state, the cover layer 21 is bonded to the substrate layer 11 and thus seals at least partially from the top the microfluidic structures on the substrate layer 11. The substrate layer 11 comprises a mixing chamber 31 for mixing at least one sample with at least one reagent, dosing chambers 32 for delivering a defined volume of samples to the mixing chamber 31, a reagent channel 37 for delivering at least one reagent to be mixed with the sample, wherein the mixing chamber 31 also serves as detection chamber. The volume defined by the sample dosing chambers 32 is about 200 nL. Two microchannels 33, 34 are connected to each dosing chamber: one sample inlet channel 33 allowing a sample to fill the sample dosing chamber; one liquid decanting channel 34, defining where the sample dosing chamber 32 starts and the sample inlet channel 33 ends, and allowing excess sample to be guided to a waste chamber 38. At about the opposite side, the sample dosing chambers 32 comprise a microfluidic valve 35. The microfluidic valve 35 is a geometric valve better visible in the enlarged view of figure 2. At this position the sample flow will temporarily stop and any excess of sample will be deviated to the decanting channel 34 and through decanting channel 34 to a waste chamber 38. The volume of the mixing chamber 31 is about 25 µ*L* and it does not need to be entirely filled in order for reaction and detection to take place.

A sample delivery channel 36 extending from the valve 35 to the mixing chamber 31 delivers the sample dosed by the sample dosing chamber 32 to the mixing chamber 31.

A sample delivery channel 36 extending from the valve 35 to the mixing chamber 31 delivers the sample dosed by the sample dosing chamber 32 to the mixing chamber 31.

The reagent channel 37 delivers the at least one reagent to the mixing chamber 31.

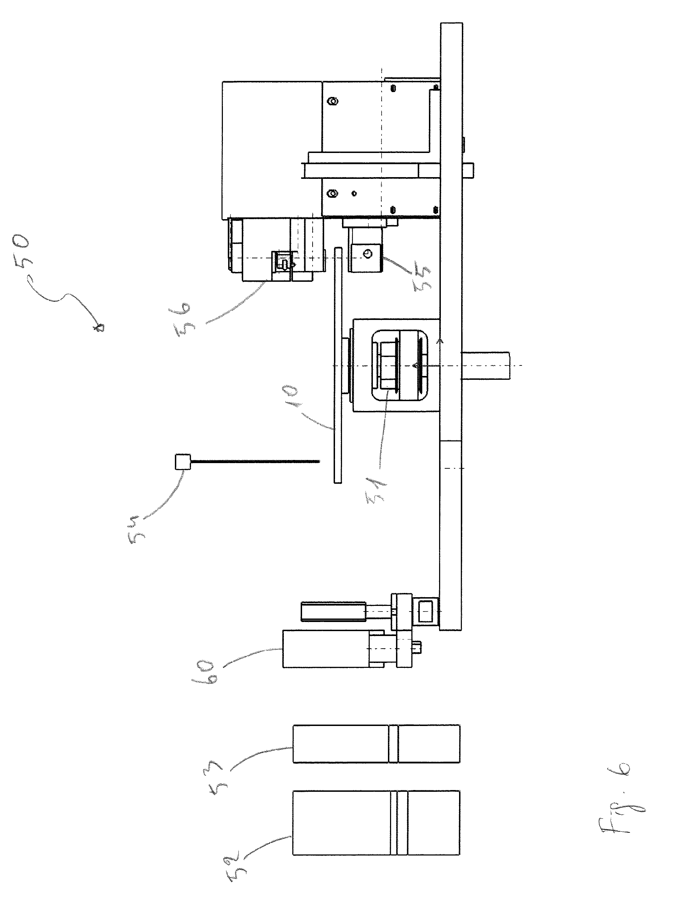

The liquid processing unit 30 further comprises a reagent inlet chamber 40 connected to the reagent channel 37 for introducing a defined volume of at least one reagent. Reagents are introduced into the reagent inlet chamber 40 via an access port or hole 41 on the cover layer 21 by means of a pipetting unit, comprising e.g. a needle 54 as schematically shown in figure 6.

The liquid processing unit 30 further comprises sample inlet chambers 39 connected to the sample inlet channels 33 for introducing a defined volume of at least one sample. Samples are introduced into the sample inlet chambers 39 via access ports or holes 42 by means of a pipetting unit, comprising e.g. a needle as schematically shown in figure 6. Also shown in figure 1 are access ports 43, 44 for air, functioning as vents for the mixing chamber 31 and the waste chamber 38 respectively.

The presence and/or quantitation of any analyte of interest is determined by photometric detection directly in the mixing chamber 31 after or during the mixing between the at least one sample and the at least one reagent.

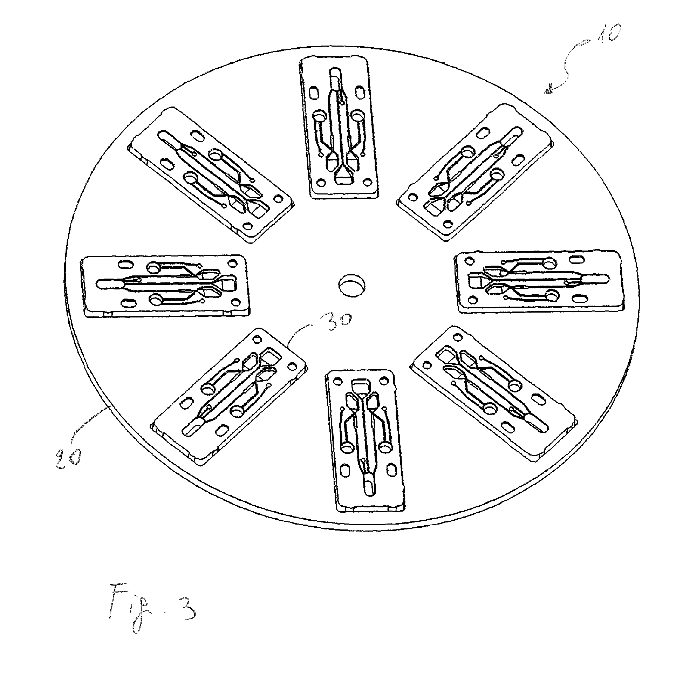

Figure 3 shows schematically an analytical device 10 comprising a plurality of liquid processing units 30 as those in figure 1 symmetrically coupled to a disc-like device body 20. For clarity, cover layers 21 are not shown. In this case the device body 20 has frame-like compartments adapted to releasably receive liquid processing units 30, wherein the liquid processing units 30 are disposable and the device body 20 is reusable, e.g. steadily coupled to a rotor 51, shown in figure 6.

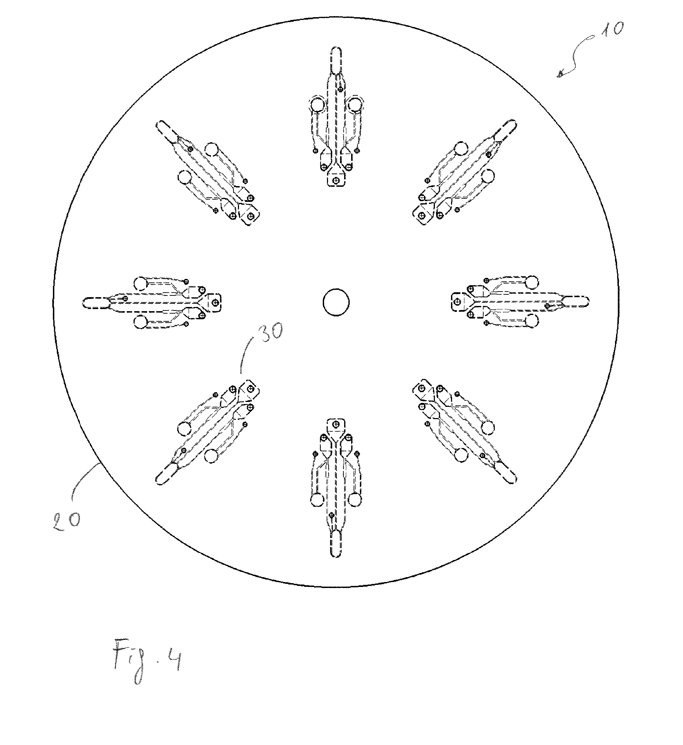

Figure 4 shows schematically an analytical device 10 comprising a plurality of symmetrically arranged liquid processing units 30 which are integral part of the disc-like device body 20. The microfluidic structures of the liquid processing units 30 are created on the upper surface of the device body 20. This means that the device body 20 serves also as substrate layer 11 for a plurality of liquid processing units 30. A cover layer 21 is in this case bonded to the device body 20 and thus seals at least partially from the top the microfluidic structures on the device body 20. Depending on the assay and the detection method used, either the device body 20 or the cover layer 21 or both are transparent at least in correspondence of the mixing chambers 31. In this case the entire analytical device 10 is disposable.

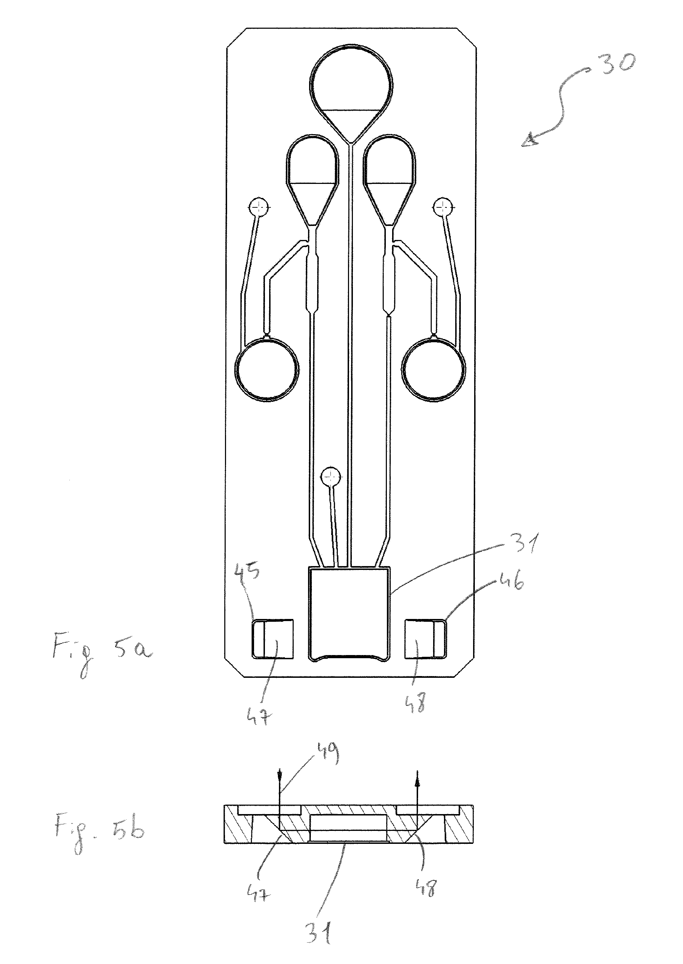

Figure 5a shows a variant of the liquid processing unit 30 of figure 1 adapted for in-plane detection. The cover layer 21 is for clarity not shown. The difference with the liquid processing unit 30 of figure 1 is the adapted shape of the mixing chamber 31 and two optical structures 45, 46 comprising reflective edges 47, 48 respectively.

Figure 5b is a side view of the liquid processing unit of figure 5a showing the arrangement of the optical structures 45, 46. The reflective edges 47, 48 form an angle of 45° relative to the plane of the analytical device 10. A light beam 49 is deflected to 90° by edge 47 and thus guided through the mixing chamber 31 before being deflected again to 90° by edge 48 out of the device body 10. Reflection is in this example based on Total Internal Reflection (TIR).

Figure 6 shows schematically a system 50 for the analysis of chemical or biological samples comprising an analytical device 10 as that of figures 3 or 4, a rotor 51 for rotating said analytical device 10, a reagent rack 52 for receiving reagent containers, a sample rack 53 for receiving sample containers, a needle 54, part of a pipetting unit (not shown), for introducing samples and/or reagents into said analytical device 10, a washing unit 60 for washing the needle 54 of the pipetting unit, an optical detection unit 55 for detecting in the mixing chambers 31 of the liquid processing units 30 the result of the reaction between samples and reagents. Also shown is a light source 56 for absorbance measurement through the transparent mixing chambers 31. In this case the detection is an out-of-plane detection.

### Example of assay and method to carry out the assay

An example of diagnostic assay that can be carried out with an analytical device according to the present invention is briefly described below.

The assay concerns the quantitative determination of glucose in a liquid sample (S), such as blood plasma. The assay reagents are in this case the same of those comprised in an assay kit (Glucose HK GLUC2) used with COBAS INTEGRA^{®} systems from Roche Diagnostics. This assay is based on the reaction of the enzyme Hexokinase (HK) for catalyzing the phosphorylation of glucose by ATP to form glucose-6-phosphate and ADP. To follow the reaction, a second enzyme, glucose-6-phosphate dehydrogenase (G6PDH) is used to catalyze oxidation of glucose-6-phosphate by NADP+ to form NADPH. The concentration of the NADPH formed is directly proportional to the glucose concentration and is determined by measuring the increase in absorbance at 340 nm.

Two main reagents are used, called R1 and R2 respectively. R1 comprises: TRIS 100 mmol/L, ATP 1.7 mmol/L, Mg⁺⁺ 4 mmol/L, NADP 1 mmol/L, at pH 7.8. R2 comprises: Mg⁺⁺ 4 mmol/L, HEPES 30 mmol/L, HK (yeast) ≥130 µkat/L (≥1.2 kU/L), G6PDH (microbial) ≥250 µkat/L (≥2.2 kU/L), at pH 7.0.

An example of method to carry out the above assay comprises the steps of:
a) providing an analytical device 10 comprising a device body 20, the device body 20 comprising at least one liquid processing unit 30, the liquid processing unit 30 comprising
   - a mixing chamber 31 for mixing sample S with reagents R1 and R2, the mixing chamber 31 being transparent,
   - one sample inlet chamber 39 and one sample dosing chamber 32 for delivering a defined volume of sample S to the mixing chamber 31,
   - one reagent inlet chamber 40 and one reagent channel 37 for delivering reagents R1 and R2 to be mixed with the sample S,
   - one waste chamber 38,
b) introducing into the reagent inlet chamber 40 15 µL of R1 + 2 µL of water,
c) rotating the analytical device 10 from 0 Hz to 80 Hz with 50 Hz/sec acceleration, waiting 5 sec at 80 Hz, so that the diluted R1 is guided into the mixing chamber 31, returning back to 0 Hz with 10 Hz/sec,
d) introducing into the sample inlet chamber 39 1 µL of sample S to be analyzed,
e) rotating the analytical device 10 from 0 Hz to 45 Hz with 2 Hz/sec acceleration and maintaining for 30 sec, so that the sample dosing chamber is filled with 200 nL of sample to be analyzed while the rest is guided to the waste chamber 38,
f) increasing the rotational speed to 80 Hz with 50 Hz/sec acceleration, to let the sample in the dosing chamber 32 pass into the mixing chamber 31, returning back to 0 Hz with 10 Hz/sec acceleration,
g) introducing into the reagent inlet chamber 40 3 µL of R2,
h) rotating the analytical device 10 from 0 Hz to 80 Hz with 50 Hz/sec acceleration, waiting 5 sec at 80 Hz, so that the R2 is guided into the mixing chamber 31, returning back to 0 Hz with 10 Hz/sec acceleration,
i) running a shaking profile by inverting a repeated number of times the rotational direction between 50 Hz and -50 Hz with acceleration of 100 Hz/sec, in order to improve mixing between the sample S and the reagents R1, R2 in the mixing chamber 31,
j) measuring the increase in absorbance at 340 nm and 409 nm through the transparent mixing chamber 31 as the result of the reaction between sample S and the reagents R1 and R2, using either out-of-plane or in-plane detection.

By this method, the volumes are scaled down by a factor of 10 compared to the same assay carried out on a COBAS INTEGRA^{®} while precision, coefficient of variation and assay time are comparable.

## Claims

1. Analytical device (10) for the analysis of chemical or biological samples comprising
- a device body (20), the device body (20) comprising at least one liquid processing unit (30), the liquid processing unit (30) comprising
- at least one mixing chamber (31)for mixing at least one sample with at least one reagent,
- at least one sample dosing chamber (32) for delivering a defined volume of sample to the mixing chamber (31),
- at least one reagent channel (37) for delivering at least one reagent to be mixed with the sample,
wherein the mixing chamber (31) also serves as detection chamber.

2. Analytical device according to claim 1 wherein a plurality of liquid processing units (30) is symmetrically arranged around a central axis (22) of rotation of the device body (20).

3. Analytical device according to claim 1 or 2 wherein the liquid processing units (30) are integral part of the device body (20).

4. Analytical device according to claim 1 or 2 wherein the liquid processing units (30) are coupled to the device body (20).

5. Analytical device according to any of the preceding claims wherein the dosing chamber (32) comprises a geometrical or hydrophobic valve (35).

6. Analytical device according to any of the preceding claims wherein the sample dosing chamber (32) has a defined volume below 1 µL and the mixing chamber (31) has a defined volume below 50 µL.

7. Analytical device according to any of the preceding claims wherein the liquid processing unit further comprises a liquid decanting channel (34) connected to the sample dosing chamber (32), said liquid decanting channel (34) allowing excess sample to be guided to a waste chamber (35).

8. Analytical device according to any of the preceding claims wherein the liquid processing unit (30) further comprises a plasma separation chamber preceding in flow direction the sample dosing chamber (32).

9. Analytical device according to any of the preceding claims wherein the liquid processing unit (30) further comprises at least one reagent inlet chamber (40) connected to the at least one reagent channel (37) for introducing a defined volume of at least one reagent via a pipetting unit (54).

10. Analytical device according to any of the preceding claims wherein the device body (20) has the shape of a disc.

11. Analytical device according to any of the preceding claims wherein the movement of liquids inside the liquid processing units (30) is controlled by controlling the speed of rotation of the device body (20).

12. Analytical device according to any of the preceding claims wherein at least the mixing chamber (31) is made of a transparent material enabling optical detection through said mixing chamber (31).

13. Analytical device according to any of the preceding claims wherein detection is based on photometric methods chosen from the group of absorbance measurement, turbidimetry, luminescence, bioluminescence, chemiluminescence, fluorescence, phosphorescence.

14. Method for the analysis of chemical or biological samples comprising the steps of
a)providing an analytical device (10) comprising a device body (20), the device body (20) comprising at least one liquid processing unit (30), the liquid processing unit (30) comprising
- at least one mixing chamber (31) for mixing at least one sample with at least one reagent, the at least one mixing chamber (31) being at least partially transparent,
- at least one sample dosing chamber (32) for delivering a defined volume of sample to the mixing chamber (31),
- at least one reagent channel (37) for delivering at least one reagent to be mixed with the sample,
- at least one waste chamber (38),
b) introducing into said analytical device (10) a chemical or biological sample to be analyzed,
c) rotating the analytical device (10) at a rotational speed so that the sample dosing chamber (32) is filled with the volume of sample to be analyzed while an excess of sample is guided to the waste chamber (38),
d)increasing the rotational speed to let the sample in the dosing chamber (32) pass into the mixing chamber (31),
e)introducing at least one reagent into said analytical device (10),
f)rotating the analytical device (10) at a rotational speed so that the at least one reagent is guided into the mixing chamber (31),
g)optically detecting through the at least partially transparent mixing chamber (31) the result of the reaction between sample and the at least one reagent.

15. Method according to claim 14 further comprising the step of separating plasma from a blood sample via a plasma separation chamber preceding in flow direction the sample dosing chamber (32).

16. System for the analysis of chemical or biological samples comprising
- an analytical device (10) according to claim 1,
- a rotor (51) for rotating said analytical device (10),
- a reagent rack (52) for receiving reagent containers,
- a sample rack (53) for receiving sample containers,
- at least one pipetting unit for introducing samples and/or reagents into said analytical device (10),
- an optical detection unit (55) for detecting in the mixing chamber (31) the result of the reaction between sample and the at least one reagent.
